# EUROPEAN PATENT APPLICATION

(11) **EP 3 647 491 A1**
(43) Date of publication of application: **06.05.2020**
(21) Application number: 19205543.2
(22) Date of filing: 28.10.2019
(51) Int. Cl.: D21F 7/00, D21F 11/14, D21G 9/00, D21F 1/40, D21F 3/08, G01N 33/34

(54) **METHOD, SYSTEM, ROTATING MACHINE ELEMENT AND COMPUTER PROGRAM PRODUCT FOR MEASURING THE MOISTURE OF A FIBRE WEB IN A TISSUE MACHINE**

(30) Priority: 26.10.2018 FI 20185902
(71) Applicant: Valmet Technologies Oy, 02150 Espoo (FI)
(72) Inventor: PITKÄNEN, Tatu, 04460 NUMMENKYLÄ (FI); RÄISÄNEN, Antti, 04480 HAARAJOKI (FI)
(74) Representative: Kespat Oy

(57) **Abstract**

The invention relates to a method for measuring the moisture of a fiber web (W) in a tissue machine (10), which includes, as sub-entities, a forming section (11), a press section (12), a Yankee drier (13) and a reel (15), each of which performs one or more process stages (39.2) included in the sub-entity and where the fibre web is taken through the sub-entity via one or more rotating machine elements (41), of which one or more are equipped with a sensor assembly (24). In the method, the temperature of the fibre web is measured directly and/or indirectly in one or more sub-entities of a tissue machine by means of a sensor assembly arranged in the coating (43) disposed on the shell (31) and/or over the shell of a rotating machine element for establishing the temperature profile (21) in the cross direction (CD) of the machine, and the moisture profile (21') in the cross direction of the machine is generated on the basis of the temperature profile. The invention also relates to a corresponding system, a rotating machine element and a computer program product.

## Description

The invention relates to a method for measuring the moisture of a fibre web in a tissue machine, which includes, as sub-entities, a forming section, a press section, a Yankee drier and a reel, each of which is used for performing one or more process stages included in the sub-entity and where the fibre web is taken through the sub-entity via one or more rotating machine elements, of which one or more is equipped with a sensor assembly. Moreover, the invention also relates to a corresponding system, a rotating machine element and a computer program product.

The moisture and moisture profile of a fibre web taken through a fibre web machine can be determined, for example, by means of a scanner adapted to measure the web after the dryer section. In some rare cases, a measurement of moisture / moisture profile implemented using a corresponding principle after the press section may also be used. However, neither of these measurements can be used for determining, for example, the machine directional trend in the moisture situation of the fibre web taking place in the machine direction. In particular, it is not possible to use known measurements to determine the real-time moisture profile of the web accurately on the basis of measurements used at present. It is known that there is an opportunity to acquire information by means of various temporary measurements or to use various calculation models, which utilise information generated by the temporary measurements. In addition to a fibre web machine, moisture information on the fibre web would also be needed in the finishing sections (online) of fibre web machines and in separate paper finishing machines (offline) .

In addition to the above, for example on many smaller paper machines, no comprehensive profile measurement scanner systems are in use. This is the case especially with tissue machines. Scanner measurement would be good per se, but it is expensive and also difficult to place in the layout in a tissue machine. Moreover, its measurement accuracy can be poor due to the thin tissue web. Yet another restriction is that scanner measurement can be provided easily at the dry end only before the reel, and it is hence not possible to obtain information on the previous process stages.

International patent application publication number WO9951811 discloses a solution, where a web scanner has been replaced with a roll with a sensor assembly, which measures the moisture profile / dry weight of the web. In the system presented, moisture is measured directly from the web using a sensor assembly, which measures impedance, which is proportional to the moisture of the fibre web. So that the impedance of the sensor can be measured, there has to be a direct physical contact with the material, in other words the fibre web. However, this measurement involves several practical problems. It is challenging to implement the impedance measurement, because issues such as air humidity and the conductivity of the roll itself cause inaccuracies in the measurement. Moreover, the sensors do not remain clean on the surface of the roll, and they may also be broken. It is very challenging to measure impedance in contact via the web especially because the boundary-layer air brought by the web tends to separate the web from the surface of the roll. In this case, it is difficult to measure the impedance changes caused by the web, when there is not even any contact.

The object of the present invention is to accomplish a method, system, rotating machine element and computer program product for measuring the moisture of a fibre web in a tissue machine.

The characteristic features of the method according to the invention are presented in claim 1, those of the system in claim 12, those of the rotating machine element in claim 14 and those of the computer program product in claim 17.

In the solution according to the invention, the temperature of the fibre web is measured directly and/or indirectly. There is a mutual correlation between the temperature and moisture of the fibre web. In this case, for example, any moist places in the fibre web are seen as cooler in a temperature measurement, and any dry places are correspondingly seen as warmer. In this way, it is possible to use the temperature of the fibre web to draw conclusions about the moisture level of the fibre web. Here, moisture level can also mean, for example, the relative moisture state of the fibre web. Relative moisture state can mean, for example, the moisture level of the fibre web in the cross direction of the machine, which moisture level varies in the cross direction of the fibre web. The variation can be presented, for example, as a cross-directional moisture profile.

As a result of the invention, the sensor assembly itself does not need to be in a physical contact with the fibre web measured. Since heat is for example conducted, and more generally, heat conduction takes place, and it is possible to measure temperature through a material, the sensor assembly can be protected inside the structures of the machine component, for example in its coating. This avoids, for example, many external sources of error. As a result of this, the measurement is insensitive to fouling and other external and disadvantageous circumstances that often exist in tissue machines.

Moreover, a temperature measurement arranged in a machine component is available at all times. It enables, for example, the online adjustment of the moisture level and/or profiling of the fibre web. The method is also relatively accurate and it enables, for example, automatic feedback.

In many other respects, too, temperature measurement is an advantageous way of measuring the moisture state of the fibre web or a quantity that is proportional to it. A measurement arrangement based on direct temperature measurement is robust against, for example, inaccuracy problems caused by prior art impedance measurement. These include, for example, air humidity, and conductivity caused by a machine component, such as a roll itself. When the measurement of the moisture of the fibre web takes place through temperature measurement, the measuring sensor assembly can be protected under a roll coating. Therefore, it does not need to be in direct physical contact with the fibre web just as long as the transfer of heat from the fibre web to the sensor assembly works.

The temperature measurement is also more insensitive to boundary-layer air. Air can cause mainly "slowness" to temperature changes, but it does not still prevent the measurement, because heat is transferred even via air, although maybe slightly more slowly. Moreover, if necessary, it is also possible to make grooves on the surface of a rotating machine element such as a roll. In this case, the air flow can be controlled better than previously.

An embodiment of the invention is temperature measurement taking place in the cross direction of the machine and integrated into machine components, such as rotating machine elements and/or static machine components adapted into contact with a web or fabric, and the moisture state of the fibre web or other quantity that is proportional to the moisture state, determined and/or determinable from it, more generally, that can be established based on it. On the basis of the temperature measurement, it is possible to generate, for example, the cross-directional temperature profile of the machine, and on the basis of it to determine and/or approximate the cross-directional moisture profile of the fibre web. In addition to a tissue machine, the invention is applicable, for example, in a fibre web machine, paper finishing machine and pulp drying machine. Other additional advantages achieved with the method, system, rotating machine element and computer program product according to the invention become apparent from the description, and the characteristic features are set forth in the claims.

The invention, which is not restricted to the embodiments presented below, is described in more detail by making reference to the enclosed drawings, in which:
- Figure 1: shows a rough diagrammatic view of an example of a fibre web machine,
- Figure 2: shows an example of a machine component equipped with a temperature sensor assembly as a rotating machine element, which machine element can be utilised in the invention,
- Figure 3: shows an example of a tissue machine,
- Figure 4: shows the example of the fibre web machine shown in Figure 1 as a diagrammatic view in principle, and the monitoring and control system belonging to it,
- Figure 5: shows an example of information formed by means of temperature measurement,
- Figure 6: shows an example of a sensor assembly of a machine component with temperature sensors as a cross section, for example in a temporary installation, and
- Figure 7: shows a second example of a sensor assembly of a machine component with temperature sensors as a cross section.

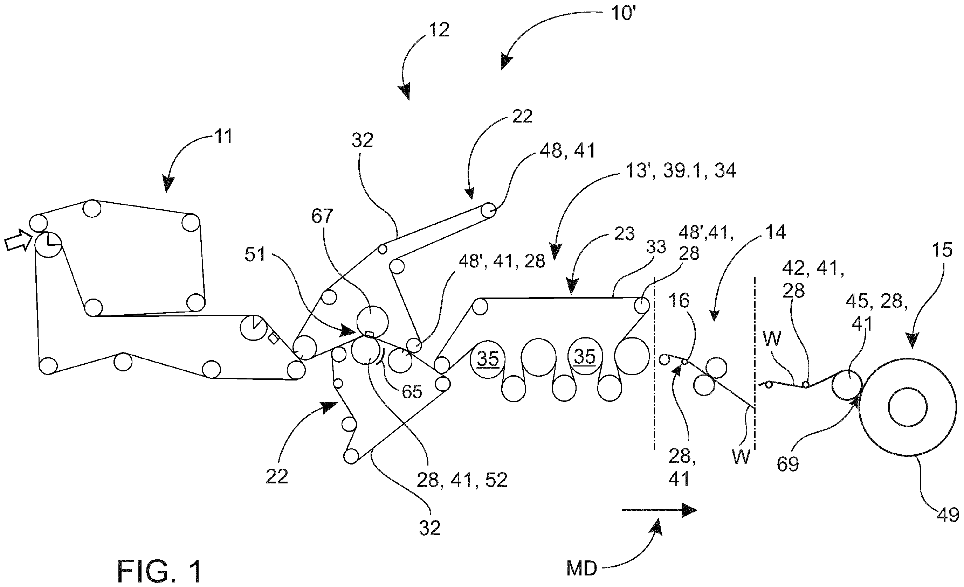

Figure 1 shows a rough diagrammatic basic view of a fibre web machine 10'. According to an embodiment, a fibre web machine 10' may include a paper finishing section 14, but this can be implemented, at least for some parts, as a separate paper finishing machine. A sub-entity 14 now shown in Figure 1 at the end of the fibre web machine 10' when viewing it in the machine direction MD corresponds to a paper finishing machine and thus to a paper finishing section.

Generally, a fibre web machine or paper finishing machine 10' includes one or more sub-entities 11 - 15. A fibre web machine 10' may include consecutive sub-entities in the direction of travel of the fibre web W, or web for short, in other words in the machine direction MD (starting from the left edge of Figure 1): headbox (not shown in Figure 1), web forming section 11 (more generally, forming section), press section 12, dryer section 13', possible finishing section 14, and reel 15. Naturally, there may be other sections, too, between the sections 11 - 15. In this way, the sequence shown is not intended to limit the invention in any way. In the finishing section 14 following the dryer section 13', such as also in a separate paper finishing machine, there can be one or more finishing devices, of which Figure 1 shows just a sizer as an example. More generally, finishing can comprise, for example, calendering, coating, surface sizing and/or second drying shown in Figure 1, which are just some examples of the process stages of finishing before the reel 15 that can be mentioned here. Moreover, processes related to paper finishing machines can also include unwinding, slitting-winding and/or rewinding.

One or more process stages belonging to a sub-entity 11 - 15 are performed on each sub-entity 11 - 15. The forming section 11 is used for forming the fibre web W, the press section 12 is used for pressing water out of the fibre web W in one or more stages, the dryer section 13' is used for drying the fibre web W, the finishing section 14 is used for possible finishing of the fibre web W, and the reel 15 is used for reeling the fibre web W onto a machine roll 49, in other words for reeling.

At least some of the sub-entities 11 - 15 of the fibre web machine 10' contain one or more machine components 44, examples of which are shown in Figures 1, 3 and 4. The fibre web W is taken, or led, through sub-entity 11 - 15 via one or more machine components 44. More generally, the machine components 44 are used for influencing the fibre web W and, on the other hand, the fibre web W also influences them. Here, influencing may mean, for example, the guiding of the travel of the fibre web W in a sub-entity 12 - 15 through the sub-entity 12 - 15.

Moreover, influencing may also mean, for example, the directing of a thermal effect on the fibre web W on the machine component 44, via which it travels, for example to dry and/or profile the fibre web W. The machine component 44 of the sub-entity 12 - 15 may also be such that the fibre web W influences it directly or indirectly, in other words secondarily. In this case, the heat of the fibre web W may be transferred either directly or indirectly, in other words secondarily, to the machine component 44, in which case it heats up (or cools down). This influencing is hence transfer of heat.

The influencing may also be direct. In this case, the fibre web W travels via a machine component 44, such as a drying cylinder 35, web lead roll 16 or reel-up drum 45, being in direct contact with it. The influencing may also be indirect, in other words secondary. In this case, the heat of the fibre web W is transferred to a machine component 44, such as to a lead roll 48' of a fabric cycle 22, 23 or to a press roll 52 belonging to a press nip 51, for example through a fabric 32, 33 or even via it transmitted by a fabric 32, 33. Hence, the fibre web W does not necessarily even have to travel via the machine component 44 to influence it. In this case, the fibre web W has influenced the fabric 32, 33, in other words been in physical contact with it, at some earlier stage of the fabric cycle 22, 23. Another alternative in indirect, or secondary, transfer of heat is that there is a fabric 32 between the fibre web W and the machine component 44. Here, the transfer of heat from the fibre web W through the fabric 32 to the machine component 44 takes place more efficiently than in an embodiment, where the fibre web W does not travel via the machine component 44, but only the fabric 32 does that, to which fabric 32 the heat of the fibre web W has first transferred and from which the heat is then transferred to the machine component 44. What is hence measured in this case is the temperature of the fabric 32, 33, which has been influenced by the web W earlier in the fabric cycle 22, 23.

One or more of the machine components 44 can be rotatable machine elements 41. Some examples of rotating machine elements 41 are rolls and cylinders 16, 35, 35', 42, 45, 48' that are in contact with the web W or that otherwise influence the web W indirectly, or secondarily. At least one fabric 32, 33 can be arranged to travel via the rolls and cylinders 35, 48', 48, as is the case, for example, in the sub-entities 11 - 13'. The fabrics 32, 33 circulate in fabric cycles 22, 23 arranged for them. The fabric 32 can be located between the web W and the machine component 41, as is the case, for example, in the press section 12. On the other hand, the web W can be located between the fabric 33 and the machine component 41, as is the case, for example, in the dryer section 13'. The sub-entity 14 can also be without a fabric cycle, at least in part. This is the case in the application example of the sizer 14', in other words the sub-entity 14 and also the reel 15. In this case, the machine element 41, in other words the rolls of the sizer, and a spreader roll 42 and reel-up drum 45 on the reel 15 are in direct contact with the web W. One or more of the machine elements 41 is equipped with a sensor assembly 24.

There are now rotatable machine elements 41 for example in the press section 12. There, two rolls 67, 52 placed against each other together form a press nip 51, via which the fibre web W now travels supported on two sides by fabrics 32. There can be one or more press nips 51 in the press section 12.

Furthermore, there are rotatable machine elements 41 also in the dryer section 13', 13. The dryer section 13', 13 typically includes elongated cylindrical machine components. Such machine components shown in Figure 1 are overhead drying cylinders 35, with which the fibre web W taken via them is dried supported by a fabric 33, and lower turn rolls 64, which may also be provided with vacuum. The cylinders 35 and rolls 64 can be grouped into dryer groups. In this case, the dryer section 13' includes multiple consecutive dryer groups to perform drying. A dryer group can be a single tier or double tier dryer group. In Figure 1, the dryer group is composed of alternate drying cylinders 35 arranged on different levels and of vacuum rolls functioning as turn rolls 64, via which a drying fabric 33 is adapted to travel as an endless loop while supported in order to transport and support the web W. A dryer group is defined, for example, by drying fabric cycles 23. There can also be drying implemented using a corresponding principle in the finishing section 14 or paper finishing machine as a single or multiple unit processes, for example after surface sizing 14' and/or coating as a post-drying relating to them. There, cylinder drying can be carried out even on just one drying cylinder, which in this case in itself forms the dryer group with its fabric, which circulates in a fabric cycle arranged for it. In this case, the dryer group may even be without turn rolls. The dryer group may also be a double tier dryer group. It can generally be said that the fabric cycles 22, 23 are arranged to support and draw the web W.

The dryer section 13' of a fibre web machine 10' may also have, in addition to a mere cylinder dryer section, also one or more vertical and/or horizontal impingement dryers (not shown). These also have their own fabric cycles with fabrics. In this case, impingement drying is typically performed on the dryers at the beginning of the dryer section 13', which is then followed by more conventional cylinder drying with heated drying cylinders 35.

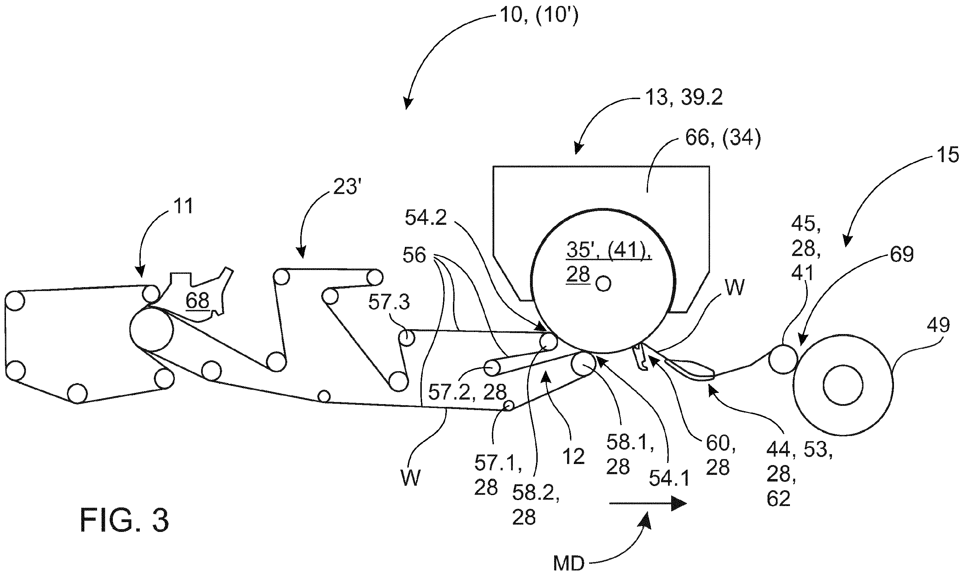

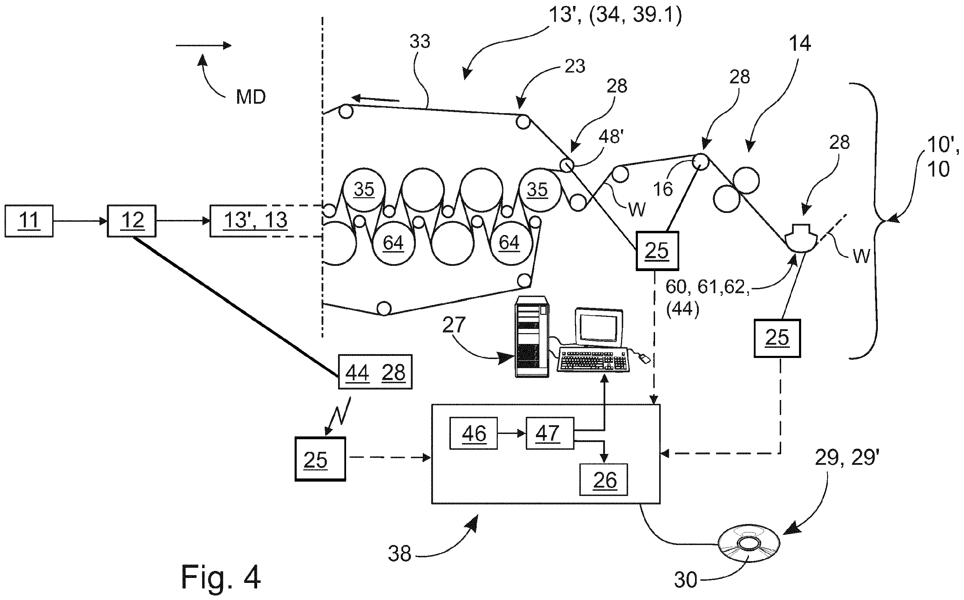

The dryer section 13' can be followed by a possible finishing section 14 in a manner known per se. The finishing section 14 is shown in Figures 1 and 4 in part only and in a rough diagrammatic view. The finishing section 14 can include one or more handling entities, in other words process stages with their equipment, performed on the fibre web W. The handling entities can be divided further into one or more unit processes. A finishing section 14 can be a fixed part of the machine line (online), in other words immediately after the fibre web machine 10', as shown in Figures 1 and 4, or also a separate sub-entity of its own (offline). In this case, the dryer section 13 of a fibre web machine 10', particularly of a tissue machine 10 (Figure 3), can be directly followed by, for example, a reel 15, with which the fibre web W is formed in at least one stage into a roll 49 before its possible finishing (further processing). Naturally, there may be other sections, too, between the sub-entities. In this way, the sequence shown or the process stages performed and/or their unit processes are not intended to limit the invention in any way. The finishing section 14 following the dryer section 13' can comprise process stages, in other words handling entities, such as various types of calendering, coating stages, surface sizing stages, and unit processes related to these - moistening, drying and/or profiling stages - which are to be mentioned in this conjunction as just some examples of the finishing section 14 before the reel 15 or of a separate paper finishing machine. There can be one or more of the same process stages in the finishing section 14, depending on the product manufactured by the machine in question or on the product manufactured in each individual case. Some of the process stages placed in finishing (such as some of the coating processes) can even be omitted, if the end product manufactured does not require it.

At least in some of the sub-entities 13', 13, 14, one or more process stages 39.1, 39.2 can be performed on the fibre web W. The process stages 39.1, 39.2 can include, for example, drying and/or profiling of the fibre web W as one or more unit processes belonging to the sub-entity 13', 13, 14. It is to be noted that drying, too, can be considered profiling of the fibre web W, especially in the finishing section 14. Process equipment accomplishing drying can include, for example, dryers, which may be contact dryers, such as cylinder dryer, in other words dryer section 13'. The dryers can also be contactless, such as IR, suspended, turnfoil, induction and air dryers. Process equipment accomplishing profiling can include, for example, moistening and calendering equipment and presses. Of course, dryers can also be used for profiling the fibre web W, as was stated above.

The text that follows describes the method according to the invention for measuring the moisture of the fibre web W in a tissue machine 10, which measurement is carried out by means of a sensor assembly 24 arranged in a machine component 44 adapted in one or more sub-entities 12, 13, 15, more specifically, in a rotating machine component 41, which sensor assembly 24 is used for measuring a quantity that is proportional to the moisture of the fibre web W. In the method, in one or more sub-entities 11 - 15 of the tissue machine 10, the sensor assembly 24 arranged on the shell 31 of a rotating machine element 41 and/or on the coating 43 disposed on the shell 31 is used to measure the temperature of the fibre web W for forming a cross-directional CD temperature profile 21 of the machine. Temperature can be measured directly from the fibre web W. Moreover or alternatively, the temperature of the fibre web W can also be measured indirectly. In this case, it can take place, for example, via a fabric 32, 33. In it, heat is transferred from the fibre web W first to the fabric 32, 33. From the fabric 32, 33, heat may be transferred through it. Furthermore, heat may be transferred with the fabric 32, 33 in the fabric cycle 22, 23 further to such a point where the fabric 32, 33 is no longer in contact with the fibre web W and where the measurement is taken.

There is a correlation between the temperature and moisture of the fibre web W. In this case, the temperature measurement 28 of the fibre web W also gives an understanding of the moisture state of the fibre web W. Hence, in accordance with the method, the cross-directional CD moisture profile 21' of the fibre web W is established on the basis of the temperature profile 21 established by the temperature measurement 28 carried out.

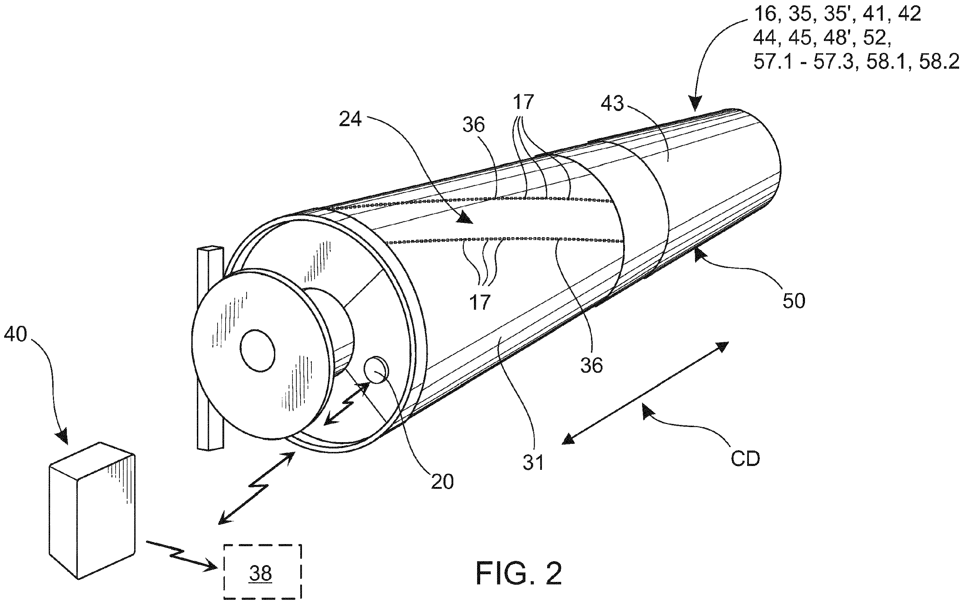

In accordance with an embodiment, the temperature of the fibre web W is measured by means of a sensor assembly 24, which is arranged in a non-oscillating manner, that is, in a way that does not move back and forth in the cross direction CD of the machine, in a machine component 44, particularly, in a rotating machine component 41, belonging to or adaptable to a sub-entity 12 - 15, for example one shown in Figure 2. For this, the machine component 44, more specifically, a rotating machine element 41, which influences the fibre web W or which is influenced by the fibre web W, directly or indirectly, is equipped with a sensor assembly 24 that measures temperature. The sensor assembly 24 includes one or more temperature sensors 17 arranged in the machine component 44 in the cross direction CD of the machine, in other words in the longitudinal direction of the machine component 44. In other words, the sensor assembly 24 is in this case arranged to carry out the measurement primarily simultaneously in the entire cross direction CD of the machine. As compared to, for example, scanning and thus oscillating measurements, this enables the generation of up-to-date temperature information and hence moisture state information generated from it from over the primary cross direction CD of the entire machine, in other words in the lateral direction of the fibre web W. In other words, the measurement and the results derived from it can in this case also be mainly real-time.

According to an embodiment, such a non-oscillating machine component 44 may be, as mentioned above, for example, at least one rotating or rotatable machine element 41. An example of one is shown in Figure 2. At least in some sub-entities 12, 13, 15 of the fibre web machine 10', and more particularly, of the tissue machine 10, there may be one or more machine elements 41 with a sensor assembly. In a rotating machine element 41, the sensor assembly 24 that measures temperature may be, for example, on the shell 31 of the machine element 41 and/or in a coating 43 adapted over the shell 31.

The sensor assembly 24 may be composed of any sensors 17 that measure temperature directly or indirectly. The sensor assembly 24 includes one or more temperature sensors 17. The temperature sensors 17 may be adapted, for example, on the shell 31 of the machine element 41 and/or under the roll coating 43, in other words on the surface of the shell 31 and/or in a coating 43 adapted over the shell 31 and/or, in some situations such as ones involving temporary measurement, also over the roll coating 43. Typically, there is one or more coating layers, more generally a coating 43, over the sensor assembly 24. The sensor assembly 24 can hence be installed on the machine element 41 in connection with its coating, for example. While the sensor assembly 24 is under the coating 43, in other words inside the coating 43, like for example between coating layers, the sensor assembly 24 is protected. While like this, the temperature of the fibre web W is measured by means of the sensor assembly 24 arranged in the machine component 44 so that the sensor assembly 24 itself and the fibre web W do not touch each other, in other words they have no physical contact. In this case, the sensor assembly 24 is protected against, for example, fouling and breaking, more generally against detrimental effects outside the machine component 44.

According to an embodiment, the temperature of the fibre web W is measured by means of a sensor assembly 24 arranged in the machine component 44 so that the transfer of heat taking place from the machine component 44 to the sensor assembly 24 is prevented, concerning, for example, heat led to the machine component 44 and/or generated in the machine component 44. The temperature of the fibre web W is hence measured by means of the sensor assembly 24, which is arranged in the machine component 44 so that the process heat brought to the machine component 44 and/or generated in the machine component 44 does not essentially influence the measurement of the temperature of the fibre web W. In this case, the heat and/or cooling generated in the machine component 44 itself / heat and/or cooling brought to it cannot influence the temperature measurement 28 performed on it.

In addition to the prevention of the transfer of heat referred to above, it is also possible to promote the transfer of heat taking place from the surface 50 of the machine component 41 to the sensor assembly 24, concerning the heat of the fibre web W and/or of the fabric 32, 33, 56 which was in contact with the fibre web W. In this way, the coating 43 of the machine element 41 can be adapted to be such in terms of the sensor assembly 24 that an as good transfer of heat as possible is enabled from the surface 50 of the machine element 41, more generally of the machine component 44, to the sensor 17. It is also advantageous that the heat of the fabric 32, 33 travelling via the fibre web W or the machine element 41 is transferred or the temperature is transmitted as well as possible to the sensor assembly 24. In this case, for example, the sensor assembly 24 is installed closer to the outer surface 50 of the coating 43 than the outer surface of the roll shell 31. According to an embodiment, this can be implemented so, for example, that the installation depth of the sensor assembly 24 from the outer surface 50 of the coating 43 is advantageously 5 - 45% of the thickness of the entire coating 43. This enables the measurement of temperature more reliably without disturbances from, for example, the potentially poorer thermal conductivity of the coating material.

Some possible examples of temperature sensors 17 here are temperature-sensitive semiconductors, resistive sensors or thermocouples. The sensor assembly 24 can be composed of, for example, a sensor band 36 or a series of sensors formed by one or more discrete sensors 17.

The temperature sensor band 36 can be in the machine component 44, in other words now in the rotating machine element 41 in the manner shown, for example, in Figure 2 in a spiral, in other words with a pitch, or it can also be in a straight line in the longitudinal direction of the machine element 41 or also in the circumferential direction. In this case, the sensors 17 can be disposed on the shell 31 of the machine element 41 at an even distance from each other. In this case, an area free from sensors remains between them. According to an embodiment, the sensor band 36 may even rotate around the machine element 41 in such a steep spiral that the sensor band 36 is rotated around the machine element 41 several times over. The angle of rotation of the sensors 17, more generally the sensor assembly 24, on the shell 31 of the machine element 41 may be 180 - 320 degrees, for example. When disposed in a pitched manner, the sensors 17 rotate around the shell 31 of the machine element 41 in a spiral manner at a distance from each other. The configuration of the temperature sensor assembly 24 can hence be quite free. With a spiral installation, however, the installation of the temperature sensor band 36 is easy, and it has the least impact on the strength of the coating 43 of the machine element 41. However, the installation geometry of the sensor band 36 in itself is not related to the operation of the sensor 17 or to the operation of the method to be presented below in more detail.

According to an embodiment, a separate conductor may be connected to each sensor 17 from the end of the machine element 41, or the sensors 17 may also be connected in parallel. In the embodiment shown in Figure 2, the sensors 17 included in the sensor band 36 are advantageously connected in series. The measurement signal of the sensors 17 is read from the sensor band 36 via, for example, four conductors. In this case, no complex cabling is needed. The width of the sensor band 36 can be a few millimetres, such as 5 mm, and the length as necessary.

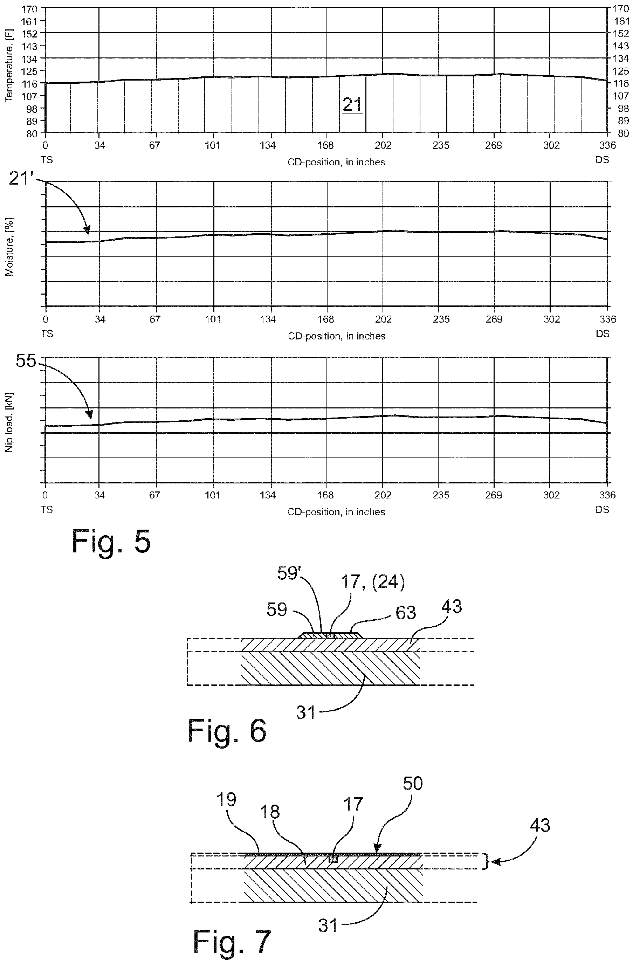

The sensors 17 that measure temperature can be intelligent in themselves. A pulse, which travels through the entire series of sensors, can be fed to the sensors 17 from the measurement electronics 40. As a result of this, each sensor 17 responds with its temperature or corresponding measurement signal generated by it when it receives an excitation impulse from the measurement electronics 40. In this case, the first sensor (closest to the measurement electronics 40) of the sensor band 36 may respond first, followed individually by each sensor 17 after it until all sensors 17 have been covered. In this way, the measurement electronics 40 may receive a measurement signal 25, which corresponds to the temperature reading, from each sensor 17 as sampled data. The sampled data can be used for generating, for example, the temperature profile 21 of the fibre web W adapted to travel via the machine element 41 (Figure 5), which temperature profile 21 has a correlation with the moisture profile 21' of the fibre web W. On the basis of the measurement data 25 generated by one or more temperature sensors 17, more generally on the basis of the temperature measurement 28, it is possible to generate the temperature profile 21 of the fibre web W in the cross direction CD of the machine, which temperature profile 21 is used for generating the moisture profile 21' of the fibre web W in the cross direction CD of the machine. Both the temperature profile 21 or just the moisture profile 21' can be displayed on a display unit 27 belonging to user interface means (Figure 4), and it can be used in the control of the circumstances of the sub-entity 12 - 15, one or more process stages or even a unit process in it and/or in condition monitoring or in the adjustment of the moisture state of the fibre web W. The embodiments related to the method are described in more detail in the description below.

Yet another way as compared to the pulse-connected/series-connected sensor band 36 described above is to use even more intelligent temperature sensors 17. In this case, each sensor 17 may have an address of its own, for example. In this case, the electronics 40 may always inquire the temperature from each sensor 17 so that the sensor, from which the temperature is inquired, is identified first with its address, then the sensor 17 responds to the inquiry for the information, and then the information is transmitted along a digital bus to the measurement electronics 40. In this configuration, the identifying address of each sensor 17 has thereby been defined for the electronics 40. When the location of each sensor 17 in the machine element 41 (in its longitudinal direction, in other words in the cross direction CD of the machine) is known, it is possible to generate the longitudinal temperature profile 21 of the machine element 41, which longitudinal temperature profile 21 correlates with the moisture profile 21' of the fibre web W (Figure 5). In a spiral installation, it is also possible to obtain the machine-directional MD temperature profile 21 of the machine element 41, in other words the circumferential temperature profile 21 of the machine element 41, and hence also the moisture profile 21' of the fibre web W in the circumferential direction of the machine element 41.

The machine element 41 may be provided with data transfer means 20 known per se for the sensor assembly 24 for delivering a measurement signal 25 generated by the sensor assembly 24 to condition monitoring included in the machine control automation 38 (Figure 4). This can be implemented, for example, with a transmitter 20 adapted at the end of the machine element 41, enabling wireless data transmission. It is used for sending the measurement signal 25 to a receiver adapted outside the machine element 41, for example one included in the measurement electronics 40. The receiver may also be provided with a transmission feature for transmitting the measurement signal 25 and/or the cross-directional temperature profile information generated by the measurement electronics 40 from it as early as in connection with the roll further to the machine control automation, to reception means 46 adapted therein. The receiver may function as a transmitter also towards the sensor assembly 24, as described above, when exciting the sensors 17 for collecting a measurement signal 25 from them. If the measurement is implemented in a static machine component 44 (Figure 4), it is of course possible to also use wire data communications between the machine component 44 and the reception means.

Figure 3 shows a rough diagrammatic view of an example of a fibre web machine, where the invention is utilised, now being a tissue machine 10. A tissue machine 10 also includes sub-entities headbox 68, forming section 11 for forming the web W, press section 12, dryer section 13, and reel 15. The components of the press section and dryer section 12, 13 can be at least partly shared by them. The fibre web is brought to the press section and dryer section 12, 13 from the web forming section 11 by a fabric 56, which travels in its cycle 23' arranged for it, for example via lead rolls 57.1 - 57.3 of the fabric 56 and also via press rolls 58.1, 58.2.

On the tissue machine 10, the fibre web W is dried by means of a Yankee dryer 13. Most typically, it is performed with a Yankee cylinder 35' included in a Yankee dryer 13, which Yankee cylinder 35' is to be heated and can hence be considered a dryer cylinder. The Yankee cylinder 35 is adapted inside a large enclosed hood 66. The fibre web W circulates on the surface of the Yankee dryer 35' at least via the area covered by the hood 66. The press rolls 58.1, 58.2 are placed against the Yankee dryer 35'. In this case, they together with the Yankee dryer 35' form the first and second press nips 54.1, 54.2. After the first press roll 58.1, the web W continues its travel while fastened to the surface of the Yankee dryer 35', and the fabric 56 diverges to travel via its lead roll 57.2 to return again into connection with the web W and the Yankee dryer 35' in connection with the second press roll 58.2. Hence, over the section between the press rolls 58.1, 58.2, the web W travels on the surface of the Yankee dryer 35'. The fibre web W is released from the surface of the Yankee dryer 35' on its outlet side by means of a crêping blade 53 or corresponding release device. The released fibre web W is then led via a slide shoe 62 to the reel 15, where it is formed into a roll 49 in the reel nip 69, taken via a reeling drum 45.

A rotating machine element 41 equipped with a sensor assembly 24 that measures temperature can be, in accordance with Figures 1, 3 and 4, for example, one or more fabric 32, 33, 56 lead rolls 48, 57.2, via which also the fibre web W is arranged to travel (or not) and/or a fibre web W lead roll 16, 57.1, 57.3, for example on the press section or dryer section 12, 13 of a tissue machine 10. On the reel 15, a specific example of a fibre web W lead roll equipped with the sensor assembly 24 that can be mentioned is, for example, a fibre web W spreader roll 42. The reeling drum 45 can be yet another individual example of a rotating machine element 41 equipped with a sensor assembly 24, located on the reel 15. The machine element 41 can also be, for example, one or more drying cylinders 35, 35' on the dryer section 13, 13' and/or on the finishing section 14. On the press section 12, the rotating machine element 41 can be, for example, one or more press rolls 52, 58.1, 58.2 that form a press nip 51, 54.1, 54.2.

Generally, a machine component 44 equipped with a sensor assembly 24 that measures temperature can be in a direct or also in an indirect, in other words secondary, heat transfer contact with the fibre web W, in other words in a contact that involves transfer of heat from the web W directly to the machine component 44 or via some component, such as via a fabric 32, 33, that transmits heat from the web W to the machine component 44. In other words, the machine component 44 equipped with the sensor assembly 24 that measures temperature is influenced by the fibre web W directly or indirectly. The influencing involves transfer of heat. The rotating machine element 41 can hence be rolls and cylinders 16, 35, 35', 42, 45, 57.1 - 57.3 in contact with the web W or, for example, rolls 48', 57.1 - 57.3, which are influenced directly or indirectly by the web W, such as a lead roll 48', 57.1 - 57.3, which defines the fabric cycle and which is influenced by the web W via or directly through the press fabric or drying fabric 32, 33, 56 that travels via it. In this case, heat is hence transferred from the web W first to the press fabric or drying fabric 32, 33, 56, from where it is transferred further to the roll 48', 57.1 - 57.3 when the fabric 32, 33, 56 circulates in its cycle 22, 23, 23' via the roll 48', 57.1 - 57.3, and the temperature can be measured by means of the sensor assembly 24 arranged in the roll 48', 57.1 - 57.3. In this case, the web W itself does not hence necessarily need to travel via the measuring roll 48', 57.2, 57.3, but what is measured here is the temperature of the fabric 32, 33, 56, which has been influenced by the web W at least at some stage of the process in the form of transfer of heat. At least with some accuracy, the temperature of the fabric 32, 33, 56 corresponds to or is in some relationship with the temperature of the web W and thereby also to and with its moisture. Heat can hence be transferred to the measured machine component 44 also via the fabric 32, 33, 56 transmitted by it or also directly through the fabric 32, 33, 56. Such indirect transfer of heat is possible, for example, on the dryer section 13, 13' or in drying by the paper finishing machine or finishing section 14, in which case temperature can be measured from the lead roll 48' of the fabric cycle indirectly via the fabric.

The lead roll 48', 57.1 - 57.3 of the fabric 32, 33, 56 or the lead roll 16 of the fibre web W, for example as a grooved roll, can be an example of the machine component 44, more specifically of the rotating machine element 41, inside the coating 43 of which the sensor assembly 24 may be located. A specific advantage of a grooved roll is, for example, the elimination of the boundary-layer air between the fibre web W and the roll surface. The sensor assembly 24 adapted to measure temperature enables the arrangement of the sensor assembly also on a grooved roll, because in this case it can be arranged inside the roll structures, such as in its coating or under it.

A roll or other corresponding rotating machine element 41 is an advantageous way of implementing temperature measurement 28 and also the measurement of the temperature profile 21 generated by it in order to generate moisture state information on the fibre web W, because it rotates at the speed of the fibre web W (unlike, for example, a static beam). Moreover, a rotating machine element 41 can also be grooved, in other words the measurement is carried out on a grooved roll or, more generally, on a rotating machine element. In this case, boundary-layer air can be minimised. As a result of this, the transfer of heat from the web W to the rotating machine element 41 can be made to be as good as possible, when the contact between the web W and the rotating machine element 41 is good. According to an embodiment, the machine component 44 can also be essentially unheated. Hence, no external heat, for example, is brought to it. In this case, its temperature is primarily influenced only by the temperature of the fibre web W and/or fabric 32, 33, 56, and hence it gives directly information on the moisture state of the fibre web W without the temperature impact caused by the machine component 44.

When implemented with the above-mentioned technology integrated into the machine component 44, the temperature measurement and the measurement of the temperature profile 21 generated by the temperature measurement 28 and the generation of the moisture state information and moisture profile 21' from it is advantageous to implement. In this case, the measurement 28 can be placed in sub-entity 12 - 15 even in several different points. The sensor assembly 24 can withstand temperatures in excess of 125°C, even 150°C and even above it. In practice, the sensor assembly 24 can be manufactured under any rubber, PU, composite or polymer coating. The implementation cost of the measurement arrangement for a single machine component 44 is in practice the cost of the sensor assembly 24 and the roll coating 43. Since the cost remains reasonable, it is hence possible to install on a sub-entity 12 - 15 even multiple machine components 44 that perform temperature measurements 28, for example in the form of rotating machine elements 41 or static beams 61 in order to determine and/or conclude, more generally, to generate moisture state information and/or moisture profile 21' from the temperature information and temperature profile 21.

As referred to above, the measurement arrangement does not necessarily require a rotating machine element 41, in other words a roll or cylinder, in which case other types of solutions for the arrangement of the measurement are also possible. A machine component 44 equipped with a sensor assembly 24 that measures temperature can also be a static, in other words stationary, non-rotating/immobile machine structure 60, as shown in Figures 3 and 4, for example. The static machine structure 60 equipped with the sensor assembly 24 that measures temperature can be, for example, a beam 61 arranged to carry out the measurement and being in contact with the web W (does not need to a scanning beam), a slide shoe 62 and/or a crêping blade 53 adapted into contact with the fibre web W and/or fabric. It is characteristic of the measuring static machine structure 60 that it is located sufficiently close with respect to the fibre web W in order to indicate essential temperature information on, for example, the pocket in question.

The temperature measurement 28 and the generation of the temperature profile 21 from it, which temperature profile 21 is proportional to the moisture state and moisture profile 21', can be carried out by measuring temperature, for example, at set time intervals, for example automatically.

The method for the monitoring and control of the operating circumstances of a fibre web machine or paper finishing machine is described below in more detail as an exemplifying embodiment referring to Figure 4. Figure 4 shows a fibre web machine 10' and machine control automation 38 with condition monitoring connected to it. Instead of the fibre web machine 10', Figure 4 could equally illustrate the tissue machine 10 shown in Figure 3. The moisture state of the web W is monitored and controlled by means of one or more machine elements 41 and/or static machine structures 60 that belong to the machine and that are, for example, rotatable in it. The shell 31 and/or coating 43 of the machine element 41 and/or machine structure 60 contains a sensor assembly 24 that measures temperature in the manner shown, for example, in Figure 2.

The machine element 41 equipped with the sensor assembly 24 that measures temperature is rotated when performing production. A measurement signal 25 is generated by the sensor assembly 24 arranged in the machine element 41 from the temperature of the machine element 41, to which temperature the measurement signal 25 generated by the sensor assembly 24 is proportional. This temperature can vary in the cross direction (CD) of the machine, in other words in the longitudinal direction of the machine element 41 and/or even the machine structure 60. The measurement signal 25 generated by the sensor assembly 24 can be saved. A cross-directional temperature profile 21 of the machine element 41 and/or the machine structure 60, for example, is generated from the measurement signal 25, and the moisture profile 21' of the fibre web can be generated further from this.

According to an embodiment, drying of the fibre web W in the cross direction CD of the machine and/or profiling of the fibre web W is controlled based on the moisture profile 21' generated based on the temperature measurement 28, for example, specifically in terms of the moisture state and moisture profile 21', hence influencing the dry matter content and/or moisture level of the fibre web W. The moisture profile 21' in particular indicates how even the cross-directional drying of the machine is. The control takes place by influencing the process stages and/or machine components, in other words by adjusting them. More specifically, one or more of the following, for example, are controlled in profiling: steam box 65 of the press section 12 (Figure 1), damper, profiling dryer 34 (such as profiling infra/suspended), such as profiling of the Yankee cylinder 35' and/or the hood 66 of the Yankee cylinder 35' and/or the press nip 51, 54.1, 54.2 in order to adjust the cross-directional CD moisture profile 21' of the fibre web W. Moisture profile measurement based on the temperature profile 21 can also be implemented as part of automatic profile adjustment, for example, in connection with the above-mentioned devices.

The temperature of the fibre web W can be measured on one or more sub-entities 11 - 15 in connection with at least one process stage 39.1, 39.2 in at least one point, but advantageously this can also be carried out in two or even more points. Advantageously, the temperature of the fibre web W can be measured before the process stage 39.1, 39.2, after the process stage 39.1, 39.2 and/or within the process stage 39.1, 39.2.

The arrangement of the measurement in multiple machine-directional MD positions enables exactly, for its part, that the sensor assembly 24 has been arranged and is arranged in a machine component 44 that is already included in / will be included in / belongs to the machine, which machine component 44 has a primary purpose other than temperature measurement. In this way, the sensor assembly 24 does not, at least as a fixed installation, necessarily require the arrangement of a specific machine component of its own in the machine for the temperature measurement 28, the adaptation of which in the machine might be particularly challenging due to, for example, the limited space in the layout. This is a prevailing condition particularly in tissue machines 10. When the temperature of the fibre web W is measured in the machine direction MD by means of the sensor assembly 24 arranged in multiple machine components 44, it is also possible to monitor the trend of the moisture profile 21' of the fibre web W in the machine direction MD. In this case, the machine-directional MD moisture level of the fibre web W can be approximated on the basis of the temperature measurement 28, and the drying capacity can be adjusted on its basis, also as a whole.

A machine component 44 arranged for the generation of the cross-directional temperature profile 21 in exact the cross direction CD of the machine is particularly advantageous, because as a component already existing in the machine and being a component that belongs to the production environment in another primary purpose, it does not require any additional components specifically for temperature measurement and for the generation of moisture information from it, the arrangement of which might even be impossible due to, for example, the limited space of the cycle. In this way, the temperature measurement performed in the machine component 44 and now arranged in the machine as a fixed installation can be referred to as a secondary function of the machine component 44, which secondary function is arranged in it in addition to the actual function intended to be performed by it (such as guiding of web or fabric, drying or sizing / surface sizing). The temperature measurement 28 for the generation of moisture information 21' can take place as a cross-directional profile measurement or also as an individual value measurement. The temperature measurement and temperature profile measurement of the web W and the moisture state information generated from it enable, for example, a better monitoring and adjustment performed on its basis of the drying of the web W and/or its finishing measures.

In addition to measurement taking place at the dry end, the result can be made more accurate by adding measurements at the wet end / before drying or within drying. As an example, temperature can be measured on the tissue machine 10 shown in Figure 3 on one or more of the following: on the press section 12 on one or more fabric 56 lead rolls 57.1 - 57.3, inside the hood 66 of the Yankee drum 35', in the area of the crêping blade 53 and on the lead roll or slide shoe 62 of the fibre web W and/or on the reel-up drum 45. This gives an understanding of the trend of the temperature profile 21 and consequently a sufficient approximation also of the trend of the moisture profile 21'. The measurement can also be used for observing the temperature profile 21 of the Yankee drum 35' at multiple points of drying inside the hood 66. In principle, the measurement can also be placed inside the Yankee drum 35'.

According to yet another embodiment, it is possible to use the temperature measurement 28 to establish a nip force profile or nip pressure profile 55 of the press nip 51, 54.1, 54.2 in the cross direction CD of the machine as a quantity that is proportional to the moisture of the fibre web W. The profile 55 can also be mainly real-time. It is also shown in Figure 5. On the basis of the temperature measurement 28, it is possible to adjust the nip force profile or nip pressure profile 55 of the press nip 51, 54.1, 54.2. Especially on tissue machines 10, the profile of the press nip 54.1, 54.2 can be seen well in the temperature profile 21, because, for example, edges loaded with too small a nip load remain more moist due to poor dewatering, and furthermore the contact with the surface of the Yankee drum 35' remains poor. Temperature profile measurement hence also gives an indication of the functioning of drying, and it can be used for improving the nip profiles and for the condition monitoring of the press.

A temperature profile sensor assembly installed on the rotating lead roll 48, 48', 16 of a fabric 32, 33 or fibre web W or on a drying cylinder 35, 35' or on the rotating machine element 41 of some process device is used for monitoring, for example primarily continuously, their temperature and/or a temperature profile 21 that is generated from it and that is proportional to the moisture state. In this way, it is possible monitor the moisture state of the fibre web W that travels via the machine component 44, which moisture state is influenced by the temperature impact caused on the fibre web at some point of the production process, which temperature impact has been accomplished by means of, for example, the drying devices and/or profiling devices of the fibre web W, such as cylinder dryer, induction device, air device or infrared device. A sensor spacing of, for example, 50 mm can be used in the cross direction CD. This indicates accurately the cross-directional CD temperature profile and consequently also the moisture profile 21' that is proportional to it. As a result of the invention, it is possible to control drying, for example, to accomplish and secure sufficient drying of the fibre web W, advantageously without excessive over-drying. More specifically, it is possible to control very accurately and advantageously automatically, for example, induction, IR, air, suspended or turnfoil dryers, which potentially also have a profiling impact on the fibre web W. As a result of the measurement and the control carried out on its basis, it is possible, for example, to optimise the functioning of the dryers and to also achieve their energy savings. By using the information generated in the measurement, for example profiling, such as the control of dampers, is also possible in order to adjust the cross-directional CD profile of the fibre web W in the desired manner.

According to an embodiment, it is also possible to directly measure the moisture profile 21' of the fibre web W in at least one point of the sub-entity 13', 13. The measurement can be carried out using a scanner after the dryer section 13', 13, for example. The moisture profile obtained from this measurement can be used as the final reference of the moisture profile. In this case, based on the measured moisture profile of the fibre web W and on one or more temperature measurements / temperature profile measurements 28 performed in various positions of the sub-entity 13', 13, it is possible to make the generation of the moisture level and/or moisture profile of the fibre web W based on the temperature measurement 28 more accurate in one or more points of drying 39.1, 39.2 and/or profiling throughout the dryer section 13, 13' or drying 39.1, 39.2 or even the entire sub-entity 13, 13'. To make this more accurate, it is also possible to use moisture profile measurement on the press section 12 or a temperature measurement arrangement placed on the press section 12, if ones are available.

According to an embodiment, when the temperature of each point and the moisture state generated from it are known, it is possible to control the functioning of the drying cylinders 35, 35', dryers 34 (such as impingement, suspended, turnfoil, IR and/or induction dryers) and/or profiling devices and/or dampers on the basis of the temperature information and moisture state information in order to control drying 39.1, 39.2 and/or the profiling of the fibre web W performed on them, in particular on dampers. In this case, it is possible to adjust and optimise the functioning of the drying means 34, and also to profile dampers. It is also possible to include profiling opportunities located before the dryer section 13' in the adjustment, such as a steam box 65 of the press section 12.

Figure 5, in turn, shows an example of information generated by means of temperature measurement 28. The figure shows the cross-directional temperature profile 21 measured by means of a sensor assembly 24 arranged in a machine element 41. A person having ordinary skill in the art understands that in reality the shape of the profile 21 can vary greatly from this. There is a location axis in the horizontal direction, in other words locations on the shell 31 of the machine element 41 in the cross direction CD of the machine, and a temperature axis in the vertical direction in Fahrenheit units. The middlemost graph, in turn, shows the moisture profile 21' generated on the basis of the temperature measurement 28. The bottom graph, in turn, shows the primarily real-time nip force profile or nip pressure profile 55 of the press nip 51, 54.1, 54.2 in the cross-direction CD of the machine, generated from the moisture profile 21' and hence from the temperature profile 21. Such information can be presented, for example, on the display unit 27 of the machine control system to the operators.

In addition to the method, the invention equally also relates to a system for measuring the moisture of the fibre web W in a tissue machine 10. The machine includes one or more sub-entities 11 - 13, 15, each of which has been adapted to perform one or more process stages 39.2 belonging to a sub-entity 11 - 13, 15. The process stages 39.2 can include, for example, drying and/or profiling of the fibre web W as one or more unit processes. The fibre web W is adapted to be taken through a sub-entity 11 - 13, 15 via one or more machine components 44, more specifically, a rotating machine element 41. One or more of the machine elements 41 is equipped with a sensor assembly 24. A sensor assembly 24 arranged in a machine component 44 adapted in one or more sub-entities 12, 13, 15, more specifically, in a rotating machine element 41, is adapted to measure a quantity that is proportional to the moisture of the fibre web W. The moisture state of the fibre web W is adapted to be generated from the quantity measured by processing means 47. In the system, on one or more sub-entities 11 - 15 the sensor assembly 24 is adapted to measure the temperature of the fibre web W, directly or indirectly. Moreover, the processing means 47 are adapted to establish the moisture profile 21' of the fibre web W in the cross-direction CD of the machine on the basis of the temperature profile 21 generated by the temperature measurement 28. The system is adapted to implement one or more of the sub-stages of the above-described method, implemented by means of, for example, a sensor assembly 24 and a computer. Machine control also includes memory means 26, for example for saving the measurements and the information generated from them, and user interface means, comprising a display unit 27, for example for viewing the measurements and the information generated from them.

In addition to the method and system, the invention also relates to a rotating machine element 41. It includes a shell 31, a coating 43 arranged over the shell 31 and a sensor assembly 24 installed, for example, in a spiral manner under or inside the coating 43 and adapted to measure temperature. The machine element 41 is used in the above-described method or system to determine and/or conclude the moisture state of the fibre web W on a tissue machine 10 on the basis of a temperature measurement 28 arranged in the machine element 41 and a temperature profile 21 generated based on the measurement.

The invention enables the online determination/approximation of the moisture state from temperature profiles 21 at different points of the sub-assembly 12 - 15 and advantageously even throughout the sub-entity 12 - 15. The invention also enables automatic adjustment of dry matter content / moisture as well as optimal control of drying and profiling. The invention can be implemented as an original installation or also as a retrofit product for a sub-entity 12 - 15 or process stage 39.1, 39.2. The invention enables the replacement of dangerous temporary measurements, which are used, for example, in the analyses and optimisation of dryer sections 13, 13'. The invention can also be implemented by way of a temporary installation, as will be described more specifically below. In this case, the temporary measurement can be installed at a desired point during a shut-down, and the production information resulting from it can be used during production for analysis.

In addition to the method and system, the invention also relates to a computer program product 29 (Figure 4) for measuring the moisture of the fibre web W in a tissue machine 10. The computer program product 29, which may be, for example, in a suitable storage medium or also downloadable over a data network or available as a service product, contains software code means 30 that implement the computer program logic stored in a storage medium 29' that is readable by a computer 27. The software code means 30 are arranged to carry out the stages of the method according to the invention for measuring the moisture of the fibre web W on the basis of a temperature profile 21 established by a temperature measurement 28 taken on one or more sub-entities 12, 13, 15 of a tissue machine 10, when the computer program is run in the computer 27.

The methods, systems and computer program logics 30 according to the invention can be arranged, for example, as part of the machine control and production control automation 38 and/or condition monitoring belonging to it. The operations according to the invention, in other words measuring, monitoring and control, are performed advantageously automatically and primarily continuously. Yet another additional advantage is that the system is learning.

What was presented above extensively were measurement arrangement examples, which are carried out, for example, in a rotating machine element 41 by means of a sensor assembly 24 most typically installed under or inside a coating 43. This requires the coating of an existing roll or cylinder or even a new machine element 41. In this case, a machine element 41 in the machine must be replaced with a coated one. This takes time, and in some positions the replacement of the roll or cylinder may be an arduous task. The coating 43 also has a cost, and it is furthermore necessary to wait for its manufacture. It is not even possible to install a coated roll in many positions, for example due to doctoring.

The sensor assembly 24 can be arranged in a machine component 44, such as a rotating machine element 41, also as a temporary installation. The advantage of a sensor assembly 24 installed temporarily is its flexibility. The sensor assembly 24 can be installed on a roll in the machine during a shut-down, and the measurements can be performed after it while the machine is operating. In a temporary installation to the surface 50 of a machine element 41, the sensor 17 senses the web W or fabric 32, 33 travelling via the surface 50 of the machine element 41 quickly, because it is on the surface 50 of the machine element 41, and there are no thermally-insulating coating layers 43 over it. On the other hand, also, when production and hence also the temporary measurement have started, the machine element 41 itself warms up relatively slowly, if it even is of a type heated/heating in the process. It therefore takes its own time before its potential heating effect extends to its surface 50. In this case, the heat caused by the machine element 41 / heat brought / generated in it does not have time to influence its surface 50 and hence the sensor 17 arranged in it. In this case, a measurement performed from the surface 50 of the machine element 41 corresponds very well to the temperature of the web W and/or fabric 32, 33.

The sensor assembly 24 can be installed during a shut-down on, for example, a web lead roll 16, and then the measurements can be performed during operation. In this case, it is possible to analyse the temperatures and temperature profiles of the web W, adjust the dryers 39.1, 39.2, collect data for models (such as DryMan) and/or also optimise the operation of other profiling devices (such as damper, thermorolls of the calender, or profiling dryers). The measurements and the analysis of the results can be carried out as a service flexibly and cost-efficiently, without waiting for issues such as coating of rolls or equipment installation. Solutions and applications described above in the description can in particular be provided as a service.

Instead of a rotating machine element 41, a temporarily-installed sensor assembly 24 can also be implemented by means of a temporarily-installed beam, which has a temperature sensor band 36. The beam is pushed into the machine to a desired location into contact with the web W or fabric 32, 33, and the beam is then influenced by the web W by way of transfer of heat. In this way, also the movable equipment can be used to flexibly measure the temperature profile of the web W, determine the moisture state of the web W based on it and generate information relating to the moisture state of the web W based on it.

In addition to the dryer section 13, 13' or finishing section 14, the temporary measurement can also be carried out at the wet end of the fibre web machine on the lead roll 48, 48' of the fabric such as a wire or press felt.

Figure 6 shows an example of a temporary sensor assembly 24 of a machine component 44 with temperature sensors 17 as a cross section. Even in a temporary installation, the sensor assembly 24 can be installed on, for example, the web lead roll 16, again in a spiral manner, on its surface 50 for temporary measurement. In this case, it withstands best, and moreover any disturbances to the travel of the web W are minimal. The sensor band 36 can be, for example, of 5 mm wide polyimide band. It contains sensor components 17 at desired intervals, such as at intervals of 50 mm. In this way, a high-resolution temperature profile measurement is obtained for analyses. If the height of the sensor component 17 so requires, an adjusting tape 59' can be installed around it. It levels out the environment of the sensor component 17 so that the component 17 does not puncture or mark the web W travelling via the roll 16. A protecting tape 63 can also be installed over the entire sensor assembly 24. This can be, for example, a Teflon or metallic tape. More generally, the sensor assembly 24 is adapted on the surface 50 of the machine element 41, and a material layer, more generally a filler 59, which is wider than the temperature sensor 17 and which levels out the surface 50 of the machine element 41, is adapted in connection with the temperature sensors 17 belonging to the sensor assembly 24, now around them, in order to level out the surface 50 of the machine element 41, in other words to create a smaller surface pressure from the point of the temperature sensors 17 to an item being in contact with the machine element 41, such as to the fibre web W.

Figure 7 shows yet another example of the sensor assembly 24 of the machine component 44, shown for example in Figure 2, with temperature sensors 17 as a cross section. Underneath the sensor assembly 24, there is one or more layers 18, which insulate, at least to some extent, the sensor assembly 24 thermally from the shell 31 of the roll. As a result of the layer 18 adapted to insulate heat, in other words to prevent transfer of heat, the heat brought to the inside of the machine component 44 and/or the heat generated in it does not disturb the temperature measurement of the fibre web W performed from the direction of the surface of the machine component 44.

Over the sensor assembly 24, there is, in turn, one or more such layers 19 that conduct heat as well as possible. In this case, heat is transferred from the web W efficiently to the sensors 17. If the coating 43 is, for example, of a single material, the layer 19 over the sensor 17 is then thinner than the layer 18 underneath it over the surface of the shell 31 of the machine element 41. In this way, by arranging the sensor assembly 24 in the coating 43, the resolution capability and speed of the measurement can be made to be the best possible, and it is possible to measure the profile of the web W, and the roll does not level out the result too much in the cross direction. More generally, the layer 18 is adapted underneath the sensor assembly 24, which is adapted to measure temperature, in order to insulate the sensor assembly 24 thermally from the shell 31 of the rotating machine element 41. Moreover, the layer 19 is adapted over the sensor assembly 24, adapted to conduct heat to the sensor assembly 24 from the surface 50 of the machine element 41. The sensor assembly 24 can be made in connection with, for example, roll coatings.

Regarding tissue machines 10, further processing machines can be mentioned as a special example of further processing. With these, smaller product rolls suitable for consumer and institutional use are typically formed out of a big machine reel. Further processing operations may include, for example, surface treatment (such as printing), unwinding, web layering lines, embossing, perforating, slitting, wind-up and/or sheeting. End products made of paper W produced with a tissue machine may be intended for household or institutional use. For example, they can be hygienic papers. Some examples include household, toilet and handkerchief papers, napkins and paper towels, cleansing tissue and industrial towels. Typically, they are consumer rolls or sheets, for example.

It is to be understood that the above description and the related figures are only intended to illustrate the present invention. The invention is hence not only restricted to the above-presented embodiments or to the embodiments defined in the claims, but several different variations and adaptations of the invention will also be obvious to a person having ordinary skill in the art, which variations and adaptations are possible within the inventive idea defined by the enclosed claims.

## Claims

1. A method for measuring the moisture of a fibre web in a tissue machine, which includes, as sub-entities, a forming section (11), a press section (12), a Yankee dryer (13) and a reel (15), each of which performs one or more process stages (39.2) included in a sub-entity (11 - 13, 15) and where the fibre web (W) is taken through a sub-entity (11 - 13, 15) via one or more rotating machine elements (41), of which one or more are equipped with a sensor assembly (24), **characterised in that**, in the method, in one or more sub-entities (11 - 13, 15) of the tissue machine (10)
- a sensor assembly (24) arranged on the coating (43) disposed on the shell (31) and/or over the shell (31) of a rotating machine element (41) is used to measure the temperature of the fibre web (W) directly and/or indirectly for generating a temperature profile (21) in the cross direction (CD) of the machine,
- a moisture profile (21') in the cross direction (CD) of the machine is formed for the fibre web (W) based on the temperature profile (21).

2. A method according to claim 1, **characterised in that** the temperature of the fibre web (W) is measured with a sensor assembly (24) arranged in a rotating machine element (41) in such a way that the sensor assembly (24) and the fibre web (W) are disposed in a contactless manner relative to each other.

3. A method according to claim 1 or 2, **characterised in that** the temperature of the fibre web (W) is measured with a sensor assembly (24) arranged in a rotating machine element (41) in such a way that
- heat transfer from the rotating machine element (41) to the sensor assembly (24) is prevented relating to heat transferred to the rotating machine element (41) and/or heat generating in the rotating machine element (41),
- heat transfer from the surface (50) of the rotating machine element (41) to the sensor assembly (24) is advanced relating to heat of the fibre web (W) and/or the fabric (32, 33, 56).

4. A method according to any one of the claims 1 - 3, **characterised in that** the rotating machine element (41) equipped with a sensor assembly (24) for measuring temperature is one or more of the following:
- a lead roll (48, 57.1 - 57.3, 16) of the fabric (32, 33, 56) and/or the fibre web (W),
- a spreader roll (42) of the fibre web (W),
- a Yankee cylinder (35') of a Yankee drier (13),
- a press roll (52, 58.1, 58.2) forming a press nip (51, 54.1, 54.2),
- a reeling drum (45).

5. A method according to any one of the claims 1 to 4, **characterised in that** the rotating machine element (41) is essentially unheated.

6. A method according to any one of the claims 1 - 5, **characterised in that**
- based on the temperature measurement (28), a nip force profile or nip pressure profile (55) of the press nip (51, 54.1, 54.2) in the cross direction (CD) of the machine is formed as a quantity that is proportional to the moisture of the fibre web (W),
- on the basis of the temperature measurement (28), the nip force profile or nip pressure profile (55) of the press nip (51, 54.1, 54.2) is adjusted.

7. A method according to any one of the claims 1 - 6, **characterised in that** temperature is measured in one or more sub-entities (11 - 13, 15) in connection with at least one process stage (39.2) in two or more points advantageously before the process stage (39.2), after the process stage (39.2) and/or within the process stage (39.2).

8. A method according to any one of the claims 1 - 7, **characterised in that** the temperature of the fibre web (W) in the machine direction (MD) is measured by means of the sensor assembly (24) arranged in multiple rotating machine elements (41) in order to monitor the trend of the moisture profile (21') of the fibre web (W) in the machine direction (MD).

9. A method according to any one of the claims 1 - 8, **characterised in that** on the basis of the moisture profile (21') generated by the temperature measurement (28), the following items of the fibre web (W) are controlled in the cross direction (CD) of the machine:
- drying and/or
- profiling, where, for example, one or more of the following are controlled: steam box (65), damper, profiling dryer (34), such as, for example, profiling of a Yankee cylinder (35') and/or a hood (66) of a Yankee cylinder (35') and/or the press nip (51, 54.1, 54.2) in order to adjust the cross-directional (CD) moisture profile (21') of the fibre web (W).

10. A method according to any one of the claims 1 - 9, **characterised in that** the machine-directional (MD) moisture state of the fibre web (W) is approximated on the basis of the temperature measurement (28), and on its basis, the drying capacity is adjusted as a whole.

11. A method according to any one of claims 1 - 10, **characterised in that** the sensor assembly (24) is arranged on the surface (50) of the rotating machine element (41) for a temporary measurement.

12. A system for measuring the moisture of a fibre web in a tissue machine, which includes, as sub-entities, a forming section (11), a press section (12), a Yankee dryer (13) and a reel (15), each of which performs one or more process stages (39.2) included in a sub-entity (11 - 13, 15) and where the fibre web (W) is arranged to be taken through a sub-entity (11
- 13, 15) via one or more rotating machine elements (41), of which one or more are equipped with a sensor assembly (24), **characterised in that**, in the system, in one or more sub-entities (11 - 13, 15) of the tissue machine (10)
- by means of a sensor assembly (24) disposed on the coating (43) arranged on the shell (31) and/or over the shell (31) of one or more rotating machine elements (41) is arranged to be measured the temperature of the fibre web (W) directly and/or indirectly for establishing the temperature profile (21) in the cross direction (CD) of the machine,
- on the basis of the temperature profile (21) by means of processing means (47) are adapted to be established a moisture profile (21') of the fibre web (W) in the cross direction (CD) of the machine.

13. A system according to claim 12, **characterised in that** the system is arranged to execute the sub-stages of the method according to one or more claims 2 - 11.

14. A rotating machine element, which includes:
- a shell (31),
- a coating (43) arranged over the shell (31) and a temperature sensor assembly (24) installed under or inside the coating (43),
**characterised in that** the machine element (41) is used in the method according to claim 1 or in the system according to claim 12 to determine and/or conclude the moisture state of the fibre web (W) on a tissue machine (10).

15. A machine element according to claim 14, **characterised in that**:
- a layer (18) is adapted under the sensor assembly (24) for preventing transfer of heat to the sensor assembly (24) from the shell (31) of the rotating machine element (41),
- a layer (19) is adapted over the sensor assembly (24) for preventing transfer of heat to the sensor assembly (24) from the surface (50) of the machine element (41).

16. A machine element according to claim 14 or 15, **characterised in that** the sensor assembly (24) is adapted to the surface (50) of the machine element (41), and a filler (59), which is wider than the temperature sensor (17), is adapted in connection with the temperature sensors (17) belonging to the sensor assembly (24) for levelling out the surface (50) of the machine element (41) at the location of the temperature sensors (17).

17. A computer program product for measuring the moisture of a fibre web in a tissue machine, **characterised in that** it comprises software code means (30) stored in a storage medium (29') readable by a computer (27), which software code means (30) are arranged to execute all of the stages of the method defined in any one of the claims 1 - 11, when the computer program is run in the computer (27).
